# EUROPÄISCHE PATENTSCHRIFT

(11) **EP 1 472 336 B1**
(45) Veröffentlichungstag und Bekanntmachung des Hinweises auf die Patenterteilung: **21.03.2007**
(21) Anmeldenummer: 03709596.5
(22) Anmeldetag: 28.01.2003
(51) Int. Cl.: C12M 3/06

(54) **VERFAHREN UND VORRICHTUNG ZUR KULTIVIERUNG VON ZELLEN IN HOHEN DICHTEN UND ZUR GEWINNUNG VON PRODUKTEN AUS DIESEN ZELLEN**
METHOD AND DEVICE FOR CULTIVATING CELLS IN HIGH DENSITIES AND FOR OBTAINING PRODUCTS FROM SAID CELLS
PROCEDE ET DISPOSITIF DE CULTURE DE CELLULES EN DENSITES ELEVEES ET DE PREPARATION DE PRODUITS A PARTIR DE CES CELLULES

(30) Priorität: 28.01.2002 DE 10204382
(43) Veröffentlichungstag der Anmeldung: 03.11.2004
(73) Patentinhaber: ProBioGen AG, 13086 Berlin (DE)
(72) Erfinder: MARX, Uwe, 13089 Berlin (DE); RIEDEL, Marco, 13187 Berlin (DE); BUSHNAQ-JOSTING, Hikmat, 10439 Berlin (DE)
(74) Vertreter: Wehlan, Helmut
(86) Internationale Anmeldenummer: PCT/DE2003/000283
(87) Internationale Veröffentlichungsnummer: WO 2003/064586

(56) Entgegenhaltungen:
- EP-A- 0 234 868
- WO-A-99/02646
- US-A- 5 081 036
- US-A- 5 510 257
- US-B1- 6 306 644
- WEATHERS P J ET AL: "AEROPONICS FOR THE CULTURE OF ORGANISMS TISSUES AND CELLS" BIOTECHNOLOGY ADVANCES, Bd. 10, Nr. 1, 1992, Seiten 93-115, XP001150080 ISSN: 0734-9750
- SHATFORD RUSSELL A ET AL: "Hepatocyte function in a hollow fiber bioreactor: A potential bioartificial liver" JOURNAL OF SURGICAL RESEARCH, Bd. 53, Nr. 6, 1992, Seiten 549-557, XP008022832 ISSN: 0022-4804
- FRIBERG J A ET AL: "CULTURE OF AMEBOCYTES IN A NUTRIENT MIST BIOREACTOR" IN VITRO CELLULAR AND DEVELOPMENTAL BIOLOGY, THE ASSOCIATION, GAITHERSBURG, MD, US, Bd. 28A, Nr. 3, PART 1, März 1992 (1992-03), Seiten 215-217, XP008011640 ISSN: 0883-8364 in der Anmeldung erwähnt

## Beschreibung

Die Erfindung betrifft ein Verfahren und eine Vorrichtung, die es erlauben, Zellen steril über lange Zeiträume in hoher Dichte zu kultivieren und die maximale Produktmenge pro Zeiteinheit aus Zellkulturen zu gewinnen.

Bis zum heutigen Zeitpunkt sind viele verschiedene Zellkulturvorrichtungen für unterschiedlichste Aufgabengebiete entwickelt worden. Ein wirtschaftlich relevantes Gebiet ist das Kultivieren von Zellen zur Herstellung von Arzneimitteln. Heutzutage werden die Zellen vor allem durch zwei grundsätzlich verschiedene Verfahren in Kultur gehalten:
1. Die Suspensionskultur in gängigen sterilen Rührkesselbioreaktoren.
2. Die stationäre Kultivierung in hohen Zelldichten, die vor allem durch das Vorhandensein geeigneter Trennmembranen möglich wurde und 1974 erstmals von Knazek et al. im Patent US 3,821,087 A beschrieben wurde. Neben Kultursystemen aus Hohlfasermembranen wurden hier auch Flachmembranen wie von Scheirer und Katinger (1985, DE 34 09 501 A1) beschrieben, eingesetzt. In den genannten Verfahren und Vorrichtungen ist jedoch die gleichmäßige Nährstoffversorgung und insbesondere die Sauerstoffversorgung problematisch. Sowohl der Versuch, dieses Problem über komplexe Verfahrensschritte mit Druckbeaufschlagung zu lösen (1989, US 4,804,628 A), als auch das direkte Einbringen von Sauerstoff in den Zellkulturraum über ein weiteres Membransystem (1986, DE 24 31 450 A1 und 1995, DE 42 30 194 A1) führten nicht zu beliebig im Maßstab vergrößerbaren Kultursystemen in denen die Zellen gleichmäßig versorgt werden können. Membranverfahren besitzen mehrere Vorteile gegenüber konventionellen Suspensionskulturen. Durch ihre Betreibung als Perfusionskulturen können sie - durch eine große Membranfläche pro Volumeneinheit - sehr hohe Zelldichten (10⁷-10⁸ Z/ml) erreichen. Außerdem sind die Zellen vor schädlichen Scherkräften geschützt, bedürfen weniger Nährstoffe und die Verfahren weisen eine höhere Produktkonzentration auf in der Erntelösung auf (Piret und Cooney, 1990 - Literaturverzeichnis hinter den Ausführungsbeispielen). Außerdem können mit Hohlfasern sowohl Suspensionszellen als auch adhärente Zellen kultiviert werden (Lipman & Jackson 1998).

Ein Problem der Hochzelldichtekultivierung ist die Maßstabsvergrößerung: Stärker noch als bei der Rührkesselfermentation ist bei Hochzelldichtesystemen die Versorgung der Zellen mit aus der Gasphase kommenden Nährstoffen wie Sauerstoff und die Abführung von Metaboliten (bevor die Konzentration lokal toxische Werte erreicht) eine Grundvoraussetzung für das Gelingen der Zellkultivierung.

Bei Hohlfaserbündelbioreaktoren, in denen die Zellen zwischen den Hohlfasern kultiviert und die Nährstoffe im Lumen der Faser transportiert werden, ist die Maßstabsvergrößerung durch die Länge der Hohlfasern limitiert. Die Länge der Hohlfasern ist jedoch durch den Verbrauch des Sauerstoffs aus den Hohlfasern begrenzt. Dadurch ist eine Maßstabsvergrößerung nur durch Parallelisierung möglich. Dies führt aber in der Praxis zu unrentablen Prozessen, d.h. die Skalierbarkeit der Hohlfaserbioreaktoren scheitert an einer adäquaten homogenen Versorgung der Zellen mit frischen Gas- und Flüssignährstofflcomponenten.

Einige kommerziell erhältliche Systeme beruhen auf Hohlfaser-Technik wie z.B. Cell-Pharm^{®}, Cellmax^{®} oder Technomouse^{®}. Alternativ dazu werden Systeme mit Flachmembranen wie CELLine^{®}, miniPERM^{®} oder OptiCell^{®} kommerziell angeboten. Allen Systemen sind die Unzulänglichkeiten eigen.

Eine andere Möglichkeit, Sauerstoff in hohen Konzentrationen in den Zellkulturraum zu bringen, ist die Verwendung von Nebel als Nährstoffquelle, wie im Patent von Weathers und Giles von 1989 (US 4,857,464 A) erstmals beschrieben wurde. Dieses Verfahren wird bis heute ausschließlich für die Kultivierung von Hairy Roots kommerziell eingesetzt. Hairy Roots sind wurzelähnliche, mit Agrobacterium rhizogenes transformierte pflanzliche Zellen, mit denen pflanzliche Sekundärmetaboliten produziert werden (Flores *et al.,* 1987). Die von Weathers und Giles vorgeschlagene Erfindung hat den Nachteil, dass Säugerzellen in hoher Dichte nicht gezüchtet werden können. Das liegt zum einen daran, dass zur Fixierung der Tierzellen am Träger ein zusammengerolltes Gitter vorgeschlagen wird, welches die Zellen tragen soll. Dieses Gitter wäre nicht geeignet, Zellen hoher Dichte, die in Einzelzellkultur gezüchtet werden, zurückzuhalten. Zumindest sind weitere Maßnahmen erforderlich, um die Haftung der Zellen zu verbessern, wie z.B. Klebemittel oder eine kovalente Verbindung mit dem Träger. Ein Ausströmen von Zellen soll in diesem Patent auch durch die Verwendung einer kissenartigen Vorrichtung verhindert werden. Nähere Angaben zu diesem Kissen werden nicht gemacht. Ein weiteres Problem dieser Erfindung stellt die Versorgung der Zellen mit Sauerstoff dar. Intakte Zellverbände zeichnen sich dadurch aus, dass sie aktive Mechanismen zum Transport von Nährstoffen inklusive Sauerstoff vom äußeren Rand des Zellverbandes zum Inneren hin besitzen.

Im genannten Patent stellt sich die Problematik der Sauerstoffversorgung nicht, da eine Kultivierung in hoher Dichte nicht vorgesehen ist. Dennoch scheint die Versorgung der Zellen mit Sauerstoff bei Verwendung der kissenartigen Vorrichtung zumindest für die Zellen im Inneren der kissenartigen Vorrichtung problematisch. Ein weiterer Nachteil ist die Produktgewinnung im US-Patent 4,857,464. Die Produkte sammeln sich zusammen mit der Nährstoffflüssigkeit im Bereich der unteren Kammer und müssen dann in einem Produktsammelbehälter getrennt werden. Auch die Zuführung und Entfernung der Zellen ist relativ kompliziert.

Das erklärt, warum die beschriebenen Nebelreaktoren - im englischen Sprachgebrauch als Nutrient mist reactors (NMB) bezeichnet - bis heute fast ausschließlich für die Kultivierung von Hairy Roots, mit der Ausnahme von Amebocyten, erfolgreich verwendet wurden (Liu et al. 1999, Wyslouzil et al. 1997, Friberg et al. 1992). Diese Amebocyten werden in den Kiemenlamellen der Limulus-Krebse produziert und werden, im Gegensatz zu den tierischen Zellkulturen im herkömmlichen Sinne, nicht als Einzelzellkulturen gezüchtet.

Liu *et al.* beschreiben die Entwicklung eines Nutrient mist bioreactors für die Produktion von Artemisinin (ein potentielles Antimalariamittel) in verschiedenen Ausführungen, basierend auf einer "Inner-loop", also auf innerer Rezirkulation, vergleichbar mit einem Airlift-Reaktor. Dafür wurde ein 2,3 1-Reaktor mit 3 Etagen Edelstahlgitter (2 mm Porengröße) als Wachstumsfläche für die Wurzeln ausgerüstet und unterschiedliche Strategien der Nährstoff-Besprühung gewählt.

Der Nebel wurde mit einem "Transducer - Ultraschall" generiert und periodisch besprüht. Die Produktivität nach 25 Tagen der so kultivierten Hairy Roots war vergleichbar mit derjenigen von submersen Kulturen nach 30 Tagen.

Wyslouzil *et al.* untersuchten die Einflüsse des Aerosoltransports und der Aerosolablagerung an derselben Art von Hairy Roots. Dabei wurde ein mathematisches Modell für die Ablagerung an den Zellen erstellt, die physischen Mechanismen der Partikelerfassung durch die Wurzelzellen (Diffusion) untersucht und schließlich das Modell mit experimentellen Resultaten verglichen. Es wurde festgestellt, dass der durch Ultraschall erzeugte Nebel mit Partikeldurchmessern zwischen 3 und 15 µm durchaus tief in ein dichtes Wurzelbett dringen kann.

Der Erfindung liegt die Aufgabe zugrunde, die Nachteile der beschriebenen Bioreaktoren zu beseitigen und neue Lösungswege zur Kultivierung von Säugerzellen hoher Zelldichte zu ermöglichen.

Die Aufgabe wurde durch ein Verfahren zur Kultivierung von Zellen in hohen Dichten und zur Gewinnung von Produkten aus diesen Zellen gelöst, welches folgende Schritte umfasst:
- Einbringen der Zellen hoher Dichte in einen Kulturraum
- Der Kulturraum besteht aus einer Vielzahl kleiner Kulturräume (Kammern)
- Die Kulturräume, hier Kammern genannt, sind von einem Raum umgeben, der hier Versorgungsraum genannt wird.
- Die Kammern sind miteinander derart verbunden, das ein Eintrag der Zellen in alle Kammern über ein Anschluss möglich ist.
- Die Kammern sind in dem Versorgungsraum fixiert, d.h. ihre Position im Raum ist festgelegt. Im Falle von zylindrischen Kammern kann die Fixierung, z.B. an den Enden der Zylinder erfolgen, sie kann jedoch auch an anderen Stellen der Kammern fixiert sein.
- Der Innenraum der Kammern ist von dem Versorgungsraum semipermeabel getrennt; permeabel sind hier alle Bestandteile außer ganze Zellen.
- Die Versorgung der Zellen, die sich im Innenraum der Kammern befinden, erfolgt über den Versorgungsraum, der mit einem variabel einstellbaren Gas/Zellkulturmediengemisch*gefüllt ist. * in Form eines Nebels oder einer Verregnung
- Die Nährstoffe (vorhanden in dem variabel einstellbaren Gas/Zellkulturmediengemisch) können anhand ihrer Größe durch die semipermeablen Wände der Kammern aus der Kammerumgebung in den Innenraum der Kammern und so zu den Zellen gelangen (z.B. durch Diffusion).
- Die Produkte können anhand ihrer Größe durch die semipermeablen Wänden der Kammern aus den Kammern in den Versorgungsraum austreten (z.B. über Diffusion).
- Die Produkte lösen sich in dem variabel einstellbaren Gas/Zellkulturmediengemisch, und können aus diesem extrahiert werden.

Überraschenderweise hat sich herausgestellt, dass durch das erfindungsgemäße Verfahren sich alle Nachteile der bekannten Zellkulturkammern beseitigen lassen und beliebig im Maßstab vergrößerbare Kultursysteme bereitgestellt werden können. Die semipermeable Trennung von Kultur- und Versorgungsraum bewirkt, dass auch Zellen hoher Dichte nicht aus den Kammern austreten. Die Versorgung der Zellen mit einem variabel einstellbaren Gas/Zellkulturmediengemisch*hat den Vorteil, dass an der semipermeablen Trennschicht Nährstoffe in die Zellkultur diffundieren. Gleichzeitig werden die Zellen im Inneren der Kammer ausreichend mit Sauerstoff versorgt. Die Zellprodukte treten an der semipermeablen Trennschicht aus der Kammer aus, vermischen sich mit der Versorgungslösung und können aus dieser extrahiert werden. * in Form eines Nebels oder einer Verregnung

Gemäß einer bevorzugten Ausführungsform wird jede Kammer so gestaltet, dass sie eine Länge von 5 mm in einer Dimension nicht überschreitet. Ansonsten sind die Formen der Kammern frei skalierbar. Das Einbringen der Zellen erfolgt mittels Trägem über ein zentrales Beschickungssystem außerhalb des Versorgungsraums. Das Aufbringen der Zellen innerhalb des Versorgungsraums entfällt somit. Demzufolge kann auf zusätzliche Fixierungsmittel wie Klebstoffe oder eine kovalente Verbindung mit dem Trägermaterial verzichtet werden. Als semipermeable Trennschicht zwischen Kultur- und Versorgungsraum werden vorzugsweise Membranen eingesetzt. Als vorteilhaft haben sich Flachmembranen oder Hohlfasermembranen erwiesen. Die Membranen bestehen aus Polymeren, z.B. aus Polysulfone, Polyethersulfone oder Polycarbonate.

Als besonders geeignet haben sich Hohlfasermembranen aus dem biokompatiblen Material Polyethersulfon erwiesen, die nach einem neuen Verfahren hergestellt werden ("Verfahren zur Herstellung von Polymer-Hohlfadenmembranen", DE 195 20 188 A1). Die Fasern, die durch dieses Verfahren hergestellt werden, können überraschenderweise ohne aufwendige Spülprozeduren für Zellkulturprozesse eingesetzt werden. Außerdem zeichnen sie sich durch eine hohe mechanische Stabilität aus und haben entsprechend große Faserinnendurchmesser. Diese Membranen bestehen aus Polymeren und wurden vorzugsweise durch ein Verfahren hergestellt, bedem ein geschmolzenes Polymer zur Bildung der Hohlfadenmembranen durch eine Extrusionseinrichtung geführt wird, wobei das Polymer unter Druck vor Eintritt in ein die Schmelze formendes Extrusionswerkzeug der Extrusionseinrichtung mit Gas beladen wird. Dabei entstehinfolge eines beim Austritt des Polymers aus der Extrusionseinrichtung in vorbestimmbaren Maße erfolgenden Druckabfalls und der damit einhergehenden Expansion des Gases im Polymer eine poröse Hohlfadenmembran.

Als Gas/Zellkulturmediengemisch wird Nebel oder eine Verregnung oder ein Gemisch aus feinen Gasblasen in Flüssigkeit eingesetzt. Der Nebel kann z.B. durch Ultraschall erzeugt werden, vorzugsweise durch ein Ultraschallschwinger mit hoher Frequenz (im Bereich 1-2 MHz).

Zur Gewinnung der Zellprodukte werden die aufgrund ihrer Schwerkraft nach unten sinkenden Tropfen in einem Gefäß gesammelt, welches sich vorzugsweise direkt unter den Kammern befindet. An dieses Gefäß ist ein Produktreservoir angeschlossen, welches sich außerhalb des Versorgungsraums befindet. Diese erfindungsgemäße Anordnung hat gegenüber dem bekannten Stand der Technik den Vorteil, dass die Produktentnahme außerhalb des Versorgungsraums erfolgt. Außerdem wird erreicht, dass das produkthaltige Medium im Gefäß unterhalb der Kammern gesammelt wird. Dadurch wird erfindungsgemäß eine Trennung zwischen produkthaltigem Medium und nichtprodukthaltigem Medium erreicht. Das nichtprodukthaltige Medium sammelt sich an den Rändern des Systems und am Boden und wird dem Gas/Zellkulturmediengemisch-Erzeuger wieder zugeführt.

Mit dem erfindungsgemäßen Verfahren können pflanzliche Zellen oder tierische Zellen (Säugerzellen) kultiviert werden.

Die erfindungsgemäße Vorrichtung besteht aus einem Kulturraum, der aus mehreren Kammern besteht, die miteinander verbunden sind und in dem Versorgungsraum fixiert sind. Der Versorgungsraum ist mit einer Einrichtung zur Erzeugung eines variabel einstellbaren Gas/Zellkulturmediengemisches*verbunden oder beinhaltet diese. Das variabel einstellbare Gas/Zellkulturmediengemisch durchströmt den Versorgungsraum und umströmt dadurch die Kammern. * in Form eines Nebels oder einer Verregnung

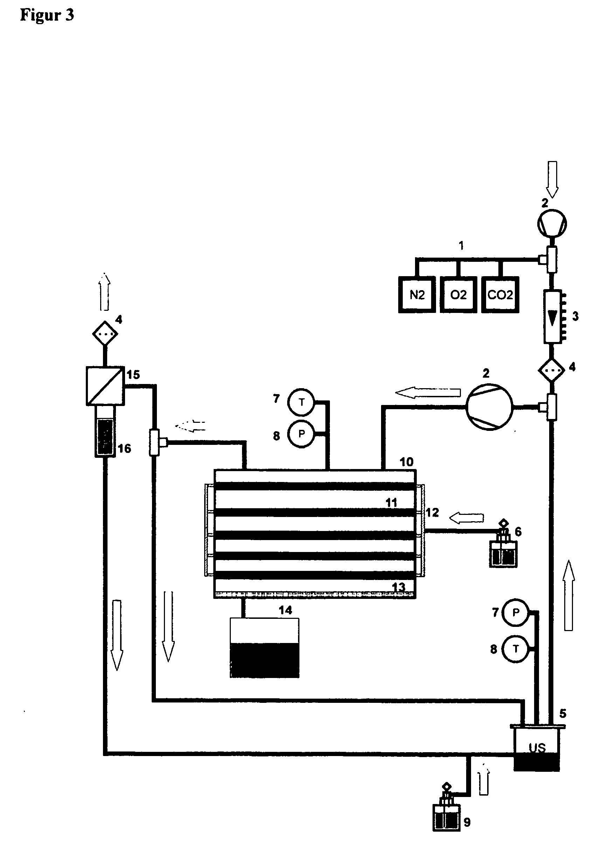

Der Kulturraum bestehend aus dem Gesamtvolumen der fixierten Kammern ist vom Versorgungsraum semipermeabel getrennt. Das Trennsystem, z.B. eine Membran, jedoch auch andere semipermeable Trennsysteme, erlaubt den Versorgungssubstraten das Austreten aus dem Versorgungsgemisch und das Eintreten in den Kulturraum und die Versorgung der Zellen. Das Trennsystem erlaubt auch das Austreten von Produkten aus den Zellkammem in die Versorgungsumgebung. Ein Beispiel für eine solche Vorrichtung ist in Figur 3 zu sehen.

Zur Abtrennung der Produkte von der Nähstoffflüssigkeit dient ein Sammelgefäß unterhalb der Kammern, verbunden mit einem Produktreservoir.

Als Vorrichtung zur Erzeugung eines Gas/Zellkulturmediengemisch dient vorzugsweise eine nebelerzeugende Kammer, z.B. ein Ultraschallschwinger mit hoher Frequenz (im Bereich 1-2 MHz). Weiterhin gehören zur erfindungsgemäßen Vorrichtung ein Rotameter zur Messung der Volumenströme der Gaszufuhr, Temperaturmessgeräte zur Überprüfung einer homogenen Temperaturverteilung, Luftfilter zur Sterilfiltration der Zu- und Abluft, Druckmessgeräte, Nebelabscheidefilter und Kondensatauffangbehälter.

Das erfindungsgemäße Verfahren und die erfindungsgemäße Vorrichtung eignen sich hervorragend für die Gewinnung von Proteinen, Wirkstoffen und Arzneimitteln.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht aus einer Kombination aus bekannten (Reaktoren mit einem Gas/Zellkulturmediengemisch*) und neuen Elementen (Trennung von Kultur- und Versorgungsraum, Kulturraum aus einer Vielzahl von Kammern, einfache Produktgewinnung), die sich gegenseitig beeinflussen und in ihrer neuen Gesamtwirkung einen Gebrauchsvorteil und den erstrebten Erfolg ergeben, der darin liegt, dass erstmals eine Möglichkeit zur Kultivierung von Zellen in hoher Dichte und zur Produktgewinnung aus diesen Zellen in beliebig im Maßstab vergrößerbaren Kultursystemen bereitgestellt wird.
* in Form eines Nebels oder einer Verregnung

Im folgenden soll die Erfindung und deren Funktion anhand von Figuren erläutert werden. Die Figuren dienen nur dem besseren Verständnis, sie sollen jedoch nicht als einzige Bauweise verstanden werden, mit der die Ansprüche realisiert werden können.

### Figur 1:

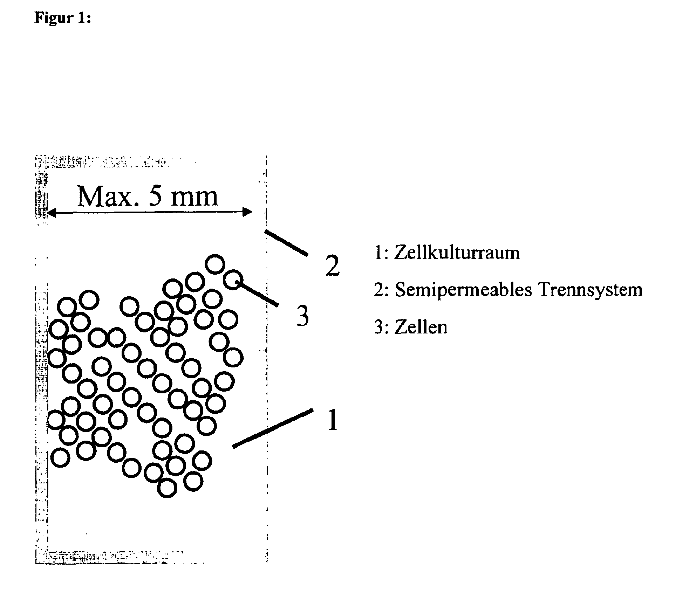

Figur 1 zeigt eine von vielen identischen Zellkulturkammern in einem Versorgungsraum, wobei der Zellkulturraum (1) einer jeden Kammer durch ein semipermeables Trennsystem (2) vom umgebenden Versorgungsraum getrennt wird und die Kammern in einer Dimension kleiner als oder gleich 5 mm sind.

### Figur 2:

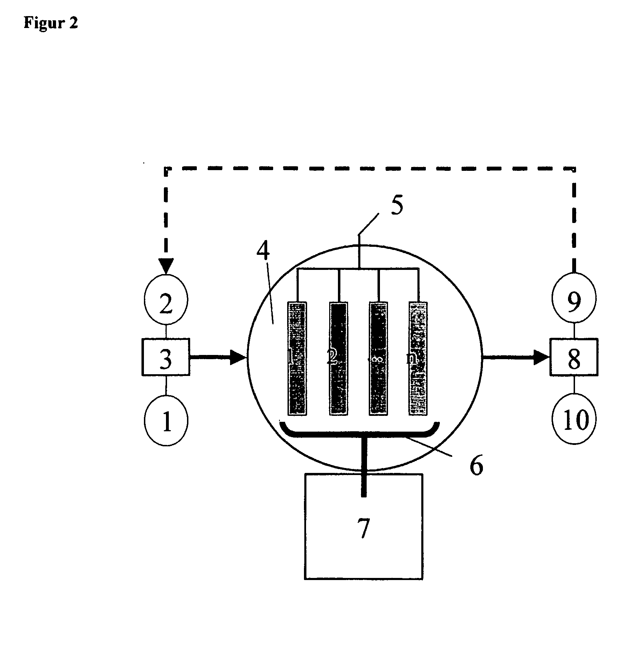

Figur 2 zeigt ein zentrales Beschickungssystem für alle Zellkulturkammern (5), eine Auffangvorrichtung für das abgetrennte Produkt (6) mit einem angeschlossenen Produktreservoir (7), eine Mischstation (3) für die Herstellung des Gas/Zellkulturmediengemisches mit Anschluss an den Versorgungsraum und eine mit dem Versorgungsraum verbundene Trennstation (8) für die Trennung des Gas/Zellkulturmediengemisches in ihre Komponenten.

Durch das zentrale Beschickungssystem werden die Zellen steril gleichmäßig auf alle Kammern verteilt.

Gleichzeitig wird der Versorgungsraum kontinuierlich mit einem entsprechenden Gas/Zellkulturmediengemisch durchströmt. Die Anordnung ermöglicht die Ablagerung von Nährlösung an den Oberflächen der Kammern. Das überschüssige Gas/Zellkulturmediengemisch strömt aus einem Ausgang in die Trennstation, wo das Zellkulturmedium vom Gas getrennt wird und bei Bedarf der Mischstation wieder zugeführt werden kann.

Zwischen der Nährlösung an der Oberfläche der Kammer und dem Zellkulturraum findet ein kontinuierlicher Austausch von Nährstoffen und gebildeten Produkten statt, so dass die Zellen kontinuierlich mit frischen Nährstoffen versorgt werden und die Stoffwechselprodukte und Produkte aus der Kammer austreten.

Die mit Produkten und Stoffwechselprodukten angereicherte Nährlösung tropft in ein Sammelgefäß (6). Von dort wird es in ein Produktreservoir (7) gesammelt. Damit ist eine einfache Trennung der Produktemte von dem Gas/Zellkulturmediengemisch gewährleistet.

Diese Vorrichtung erlaubt eine Maßstabsvergrößerung durch Vervielfältigung der Anzahl der Kammern bei Beibehaltung eines gemeinsamen Versorgungsraumes.

### Figur 3:

in der Figur 3 ist eine Vorrichtung gezeigt, die nach dem Funktionsprinzip von Figur 2 funktioniert. In dieser Vorrichtung wird ein Ultraschallverfahren (5) eingesetzt, um einen Nebel zu erzeugen. Der Nebel wird mit einer Luftpumpe (2) gefördert, durchströmt den Versorgungsraum (10) und umströmt die Kammern (11) im Versorgungsraum. Die Zellen (6), welche über eine zentrale Zelleintragvorrichtung (12) in die Kammern zu Beginn des Prozesses gebracht werden, werden über den Nebel versorgt. Die Produkte wandern aus dem Inneren der Kammern durch die semipermeable Wand (in diesem Fall eine Hohlfaser) und lösen sich in dem kondensierten Nebel an der Oberfläche der Kammern. Das Produkthaltige Kondensat tropft ab und wird im Sammelgefäß (13) aufgefangen und von dort in ein Produktreservoir (14) transportiert.

Nebel, der aus dem Versorgungsraum austritt, wird im Kreislauf wieder zur Ultraschallquelle (5) gebracht. Bei Bedarf kann frische Prozessluft gezielt in die Vorrichtung über Gasvorräte (1), Luftpumpe (2) und einem Rotameter (3) steril (durch den Filter 4) eingebracht werden. Die überschüssige (verbrauchte) Luft im System wird über einen Nebelabscheider (15) in die Fraktionen Gas und Zellkulturmedium (16) aufgeteilt und zu einer weiteren Verwertung oder einer Entsorgung geführt.

Die Erfindung soll anhand von Ausführungsbeispielen erläutert werden, ohne auf diese Beispiele beschränkt zu sein.

### Ausführungsbeispiele:

### Beispiel 1

Es wurden als Kammern Kassetten eingesetzt, die von zwei parallel zu einander stehenden Seiten mit porösen Membranen begrenzt wurden und wo der Abstand zwischen den Membranen kleiner 5 mm war. Das Gas/Zellkulturmediengemisch bestand aus vernebeltem Zellkulturmedium in einer CO₂/Raumluft-Atmosphäre.

Es wurden 6 Kammern mit einer Zellkonzentration von jeweils 21 Mio. Hybridomazellen pro ml eingesetzt.

Jeweils zwei Kammern wurden am vierten und 4 Kammern am achten Tag der sterilen Kulturführung aus dem Versorgungsraum entnommen und auf die Anzahl der Zellen untersucht.

Die Ergebnisse sind in der nachfolgenden Tabelle als Mittelwert mit Angabe der Abweichung (n=2 am Tag 4 und n=4 am Tag 8) dargestellt.

**Tabelle 1:**

| | | |
|---|---|---|
| Kulturzeit (Tage) | 4 | 8 |
| Zellkonzentration (10⁶ Z/ml) | 1,68 ± 0,40 | 36,60 ± 0,26 |

Nach einem erwarteten Fall der Zellkonzentration, bedingt durch Adaptationsprozesse der Zellen an einer neuen Umgebung auf 1,68 Mio. Zellen pro Milliliter, konnten sich die Zellen im Bioreaktor erholen, und innerhalb der folgenden 4 Tage wieder die Anfangskonzentration erreichen und überschreiten. Die Endkonzentration von 36,6 Mio. Zellen/ml bedeuten nicht nur, dass die hohe Zellkonzentration zu Beginn des Versuches erhalten werden konnte, sondern eine Erhöhung der Zellkonzentration um den Faktor 1,74 möglich war. Betrachtet man nur die Ergebnisse der Tage 4 und 8, so hat sich die Zellzahl innerhalb von 4 Tagen um den Faktor 22 erhöht.

### Beispiel 2

Dazu wurden zwei Kammern wie bereits im Beispiel 1 beschrieben eingesetzt. Das Gas/Zellkulturmediengemisch bestand aus in Raumluft vernebelter Lösung.

Die beiden Kammern wurden mit je 1,2 mg Antikörper in Lösung beschickt. Die von den Kammern abtropfende Lösung wurde fraktioniert in einem gemeinsamen Abtropfgefäß gesammelt und mittels ELISA Technik auf Produkt untersucht. Das Sammeln in einem gemeinsamen Abtropfgefäß entspricht der Mittelwertbildung. Nachfolgende Tabelle stellt die Abhängigkeit der gefundenen Produktakkumulation im Abtropfgefäß von der Zeit dar.

Es ist gelungen, innerhalb von 1,5 Stunden bereits 10% des eingesetzten Produktes getrennt vom Versorgungsstrom zu gewinnen.

Das Beispiel belegt die effektive Trennung von Produkternte und Versorgungsstrom in der erfindungsgemäßen Vorrichtung.

**Tabelle 2: Abhängigkeit der gefundenen Produktakkumulation im Abtropfgefäß von der Zeit**

| Zeit Minuten | Fraktionen (ml) | Antikörperkonzentration (µg/ml) | Gesamtmasse Antikörper (µg) |
|---|---|---|---|
| 0 | 0 | 0 | 0 |
| 5 | 1 | 2.8 | 3 |
| 10 | 2 | 2.8 | 8 |
| 15 | 2 | 2.8 | 14 |
| 20 | 2 | 3.5 | 21 |
| 25 | 2.3 | 3.5 | 29 |
| 30 | 2 | 3.5 | 36 |
| 35 | 2.3 | 4.8 | 47 |
| 40 | 2 | 4.8 | 57 |
| 45 | 2 | 4.8 | 66 |
| 50 | 2.3 | 6.7 | 82 |
| 55 | 2 | 6.7 | 95 |
| 60 | 2.3 | 6.7 | 111 |
| 75 | 6 | 7.7 | 157 |
| 94 | 8 | 8.3 | 223 |

### Literaturverzeichnis

**Piret JM, Cooney CL.** 1990. Immobilized mammalian cell cultivation in hollow fiber bioreactors. *Biotechnol. Adv.* 8: 763-783.
**Lipman NS, Jackson LR.** 1998. Hollow fibre bioreactors: an alternative to murine ascites for small scale (<1gram) monoclonal antibody production. *Res Immunol* Jul-Aug;149(6):571-6
**Flores HE, Hoy MW, Pickard JJ.** 1987. Secondary metabolites from root cultures. *Tibtech.* 5:64-69
**Liu CZ, Wang YC, Zhao B, Guo C, Ouyang F, Ye HC, Li GF.** 1999. Development of a nutrient mist bioreactor for growth of hairy roots. *In Vitro Cell Dev Biol- Plant* May-June; 35:271-74.
**Wyslouzil BE, Whipple M, Chatterjee C, Walcerz DB, Weathers PJ, Hart DP.** 1997. Mist deposition onto hairy root cultures: aerosol modeling and experiments. *Biotechnol Prog.* Mar-Apr;13(2):185-94.
**Friberg JA, Weathers PJ, Gibson DG 3rd.** 1992. Culture of amebocytes in a nutrient mist bioreactor. *In Vitro Cell Dev Biol.* Mar;28A(3 Pt 1):215-7.

### Definitionen und Abkürzungen

- Einzelzellen: Einzelzellen sind Zellen, die nicht im intakten Gewebeverband auftreten.
- Gleichmäßige Versorgung: Eine Versorgung ist erfindungsgemäß gleichmäßig, wenn sie in einer Dimensionen identisch ist und in dritten Dimension ausreichend und in der prozentualen Zusammensetzung identisch ist.
- Produkte: Produkte sind erfindungsgemäß Zellbestandteile, Viren sowie in und durch Zellen produzierte Wirkstoffe.
- Semipermeables Trennsystem: Als semipermeables Trennsystem wird erfindungsgemäß die Grenzschicht zwischen Versorgungs- und Zellkulturraum bezeichnet, die sich durch Zellrückhalt bei gleichzeitiger Produkt- und Nährstoffdurchlässigkeit auszeichnet.
- Stationäre Kultivierung: Die stationäre Kultivierung ist erfindungsgemäß eine nicht aktiv bewegte Kultivierung.
- Trägersystem: Ein Trägersystem ist erfindungsgemäß ein Medium, welches zum Einbringen und/oder Stützen der Zellen in der Zellkulturkammer eingesetzt wird. Dieses Medium kann in seinen Eigenschaften flüssig, semisolid oder auch fest sein.
- Zellen: Mit dem Begriff Zellen werden natürliche und zufällig bzw. durch Manipulation entartete Zellen jeglicher Spezies bezeichnet.
- Zellen in hohen Dichten sind erfindungsgemäß Konzentrationen von Einzelzellen in steriler Kultur von über 10⁶ Zellen pro ml.
- Gas/Zellkulturgemisch: Ein Gemisch aus Flüssigem Zellkulturmedium und einem Gasgemisch, wobei das Gasgemisch nicht nur im Medium gelöst wird, sondern als zweite Phase in einem Zweiphasengemisch vorliegt. Beispiele hierfür sind Nebel, Regen (beides Beispiele für viel Gas und wenig Flüssigkeit) oder auch Gasblasen in der Flüssigkeit (z.B. über ein Begasungsring: viel Flüssigkeit und wenig Gas).

### Bezugszeichenliste:

### Figur 2:

- 1-: Gas
- 2-: Nährlösung
- 3-: Gas/Zellkulturmedienmischer
- 4-: Versorgungsraum
- 5-: Zentrale Beschickungssystem
- 6-: Sammelgefäß
- 7-: Produktreservoir
- 8-: Gas/Zellkulturmedienentmischer
- 9-: Nährlösung mit Rückführmöglichkeit
- 10-: Gas

### Figur 3:

- 1-: Gasvorrat
- 2-: Luftpumpe
- 3-: Rotameter
- 4-: Luftfilter
- 5-: Nebelkammer bestehend aus einer Kammer mit Eingängen, Ausgängen, Gasphase, Flüssigphase und ein Ultraschallschwinger, der ein Gas/Zellkulturmediengemisch in Form von Nebel erzeugt, das über die Luftphase transportiert wird
- 6-: Zellen zum Innokulieren
- 7-: Druckmessgerät
- 8-: Temperaturmessgerät
- 9-: Mediumvorrat
- 10-: Versorgungsraum
- 11-: Kammer
- 12-: Verbindung zwischen den Kammern
- 13-: Sammelgefäß
- 14-: Produktvorrat
- 15-: Nebelabscheidervorrichtung
- 16-: Kondensatauffangbehält

## Patentansprüche

1. Verfahren zur Kultivierung von tierischen Zellen in hohen Dichten und zur Gewinnung von Produkten aus diesen Zellen, **gekennzeichnet durch** folgende Schritte:
- Einbringen der Zellen hoher Dichte in einen Kulturraum, der aus mehreren fixierten Kammern besteht und der von einem Versorgungsraum semipermeabel getrennt ist
- Versorgung der Zellen über den Versorgungsraum mit einem variabel einstellbaren Gas/Zellkulturmediengemisch in Form eines Nebels oder einer Verregnung
- Gewinnung der Zellprodukte **durch** deren Austritt an den semipermeablen Wänden der Kammern und **durch** deren Lösen in dem Gas/Zellkulturmediengemisch.

2. Verfahren nach Anspruch 1, **dadurch gekennzeichnet, dass**
a) das Einbringen der Zellen in die Kammern über ein zentrales Beschickungssystem außerhalb des Versorgungsraums erfolgt und dass der Kultur- und Versorgungsraum derart miteinander verbunden sind, dass der gleichzeitige Eintrag der Zellen in alle Kammern über einen Anschluss möglich ist und
b) die semipermeable Trennung von Kultur- und Versorgungsraum mittels Membranen erfolgt.

3. Verfahren nach Anspruch 2, **dadurch gekennzeichnet, dass** als Membranen Flachmembranen oder Hohlfasermembranen eingesetzt werden, die vorzugsweise aus Polysulfon, Polyethersulfon oder Polycarbonat bestehen.

4. Verfahren nach Anspruch 1, **dadurch gekennzeichnet, dass** der Nebel eine Tropfengröße bis 100 µm bzw. die Verregnung eine Tropfengröße von 100 bis 5000 µm besitzt.

5. Verfahren nach Anspruch 4, **dadurch gekennzeichnet, dass**
a) der Nebel durch ein Ultraschallschwinger mit hoher Frequenz erzeugt bzw.
b) die Verregnung mittels Düsen oder Duschen erzeugt wird.

6. Verfahren nach Anspruch 1, **dadurch gekennzeichnet, dass** die Zellprodukte an den Membranen austreten und unterhalb der Kammern in Gefäßen aufgefangen werden, wobei das mit Produkt angereicherte Gas/Zellkultur-Mediengemisch durch Schwerkraft von den Kammern abtropft und unterhalb der Kammern gesammelt wird.

7. Vorrichtung zur Kultivierung von tierischen Zellen in hohen Dichten und zur Gewinnung von Produkten aus diesen Zellen zur Durchführung des Verfahrens nach Anspruch 1, bestehend aus einem Kulturraum und einer Einrichtung zur Erzeugung eines Nebels oder einer Verregnung, **dadurch gekennzeichnet, dass** der Kulturraum aus mehreren fixierten Kammern besteht, die von einem Versorgungsraum, der den Nebel oder die Verregnung enthält, semipermeabel getrennt sind.

8. Vorrichtung nach Anspruch 7, **dadurch gekennzeichnet, dass** jede der Kammern die Länge von 5 mm in einer Dimension nicht überschreitet.

9. Vorrichtung nach Anspruch 7 oder 8, **dadurch gekennzeichnet, dass** sich unterhalb der Kammern Gefäße zum Auffangen der Zellprodukte befinden.

10. Vorrichtung nach einem der Ansprüche 7 bis 9, **dadurch gekennzeichnet, dass**
a) als Anschluss ein Schlauch oder eine Luer-Verbindung dient
b) sie mindestens eine nebel- bzw. aerosolerzeugende Kammer enthält
c) sie eine oder mehrere Düsen oder Duschen enthält, mit denen ein Regen erzeugt werden kann
d) sie zusätzlich Rotameter zur Messung der Volumenströme der Gaszufuhr, Temperaturmessgeräte zur Überprüfung einer homogenen Temperaturverteilung, Luftfilter zur Sterilfiltration der Zu- und Abluft, Druckmessgeräte, Nebelabscheidefilter und Kondensatauffangbehälter enthält
e) das Gefäß zum Auffangen der Zellprodukte mit einem Produktreservoir verbunden ist, welches sich außerhalb des Versorgungsraums befindet.

11. Verwendung der Vorrichtung nach einem der Ansprüche 7 bis 10 zur Kultivierung von tierischen Zellen in hohen Dichten zur
a) Gewinnung von Zellprodukten, Zellbestandteilen, Viren oder Wirkstoffen
b) Gewinnung von Arzneimitteln oder
c) Herstellung von Diagnostika.

## Claims

1. Process for the cultivation of animal cells in high densities and for the extraction of products from these cells, **characterised in that** it comprises the following stages:
- Insertion of the cells in high densities into a culture chamber that comprises several fixed chambers and is semi-permeably separated from a feeding chamber
- Feeding of cells via the feeding chamber with a variably adjustable gas/cell culture medium mixture in the form of a mist or rain
- Extraction of the cell products by their exit through the semi-permeable walls of the chambers and their dissolving in the gas/cell culture medium mixture.

2. Process according to Claim 1, **characterised in that**
a) the insertion of the cells into the chambers is carried out via a central loading system outside the feeding chamber and that the culture and feeding chamber are connected to each other in such a way that the simultaneous insertion of the cells into all chambers via one connection is possible, and
b) the semi-permeable divide between the culture and feeding chamber is effected by means of a membrane.

3. Process according to Claim 2, **characterised in that** flat membranes or hollow fibre membranes are used as membranes, preferably made from polysulphone, polyether sulphone or poly carbonate.

4. Process according to Claim 1, **characterised in that** the mist has a drop size up to 100 µm or the rain has a drop size between 100 µm and 5000 µm.

5. Process according to Claim 4, **characterised in that**
a) the mist is produced by an ultrasound oscillator with high frequency, or
b) the rain is produced by means of nozzles or showers.

6. Process according to Claim 1, **characterised in that** the cell products exit through the membranes and are collected below the chambers in vessels, whereby the product enriched gas/cell culture medium mixture drips from the chambers by the force of gravity and is collected below the chambers.

7. Device for the cultivation of animal cells in high densities and for the extraction of products from these cells by carrying out the process according to Claim 1, comprising a culture chamber and a device for the production of a mist or rain, **characterised in that** the culture chamber comprises several fixed chambers that are semi-permeably separated from a feeding chamber containing the mist or rain.

8. Device according to Claim 7, **characterised in that** each chamber does not exceed a length of 5mm in one dimension.

9. Device according to Claim 7 or 8, **characterised in that** vessels to collect the cell products are located below the chambers.

10. Device according to one of the Claims 7 to 9, **characterised in that**
a) a hose or Luer connector serves as a connection
b) it contains at least one mist- or aerosol-producing chamber
c) it comprises one or more nozzles or showers that can produce rain
d) it additionally comprises a rotameter to measure the volume current of the gas inlet, temperature measuring device to check homogenous temperature distribution, air filter for sterile filtration of the feeding and exhaust air, pressure measuring device, mist separating filter and condensate collection container
e) the vessel to collect the cell products is connected to a product reservoir, which is located outside the feeding chamber.

11. Use of the device according to one of the Claims 7 to 10 for the cultivation of animal cells in high densities for
a) the extraction of cell products, cell constituents, viruses and active cell substances
b) the extraction of medicines or
c) the production of diagnostic products.

## Revendications

1. Procédé pour la culture de cellules animales en densités élevées et pour l'obtention de produits provenant de ces cellules, **caractérisé par** les étapes suivantes :
- introduction des cellules de densité élevée dans un espace de culture qui se compose de plusieurs chambres fixées et qui est séparé d'un espace d'alimentation de manière semi-perméable,
- alimentation des cellules par le biais de l'espace d'alimentation avec un mélange de gaz/milieu de culture cellulaire réglable de manière variable sous la forme d'un brouillard ou d'une pluie
- obtention des produits cellulaires par leur sortie des parois semi-perméables des chambres et par leur dissolution dans le mélange de gaz/milieu de culture cellulaire.

2. Procédé selon la revendication 1, **caractérisé en ce que**
a) l'introduction des cellules dans les chambres s'effectue par le biais d'un système de chargement central en dehors de l'espace d'alimentation et que l'espace de culture et l'espace d'alimentation sont reliés ensemble de sorte que l'entraînement simultané des cellules dans toutes les chambres est possible par le biais d'un raccord et
b) la séparation semi-perméable de l'espace de culture et de l'espace d'alimentation est réalisée au moyen de membranes.

3. Procédé selon la revendication 2, **caractérisé en ce que** des membranes plates ou des membranes à fibres creuses qui se composent de préférence de polysulfone, polyéthersulfone ou polycarbonate sont utilisées comme membranes.

4. Procédé selon la revendication 1, **caractérisé en ce que** le brouillard possède une taille de gouttes allant jusqu'à 100 µm ou la pluie possède une taille de gouttes de 100 à 5000 µm.

5. Procédé selon la revendication 4, **caractérisé en ce que**
a) le brouillard est généré par un émetteur d'ultrasons à haute fréquence ou
b) la pluie est générée au moyen de buses ou de douches.

6. Procédé selon la revendication 1, **caractérisé en ce que** les produits cellulaires sortent des membranes et sont capturés en dessous des chambres dans des récipients, le mélange de gaz/milieu de culture cellulaire enrichi en produit s'égouttant des chambres par la force de pesanteur et étant recueilli en dessous des chambres.

7. Dispositif pour la culture de cellules animales en densités élevées et pour l'obtention de produits provenant de ces cellules pour la réalisation du procédé selon la revendication 1, constitué d'un espace de culture et d'un dispositif pour la génération d'un brouillard ou d'une pluie, **caractérisé en ce que** l'espace de culture se compose de plusieurs chambres fixées qui sont séparées par un espace d'alimentation de manière semi-perméable qui contient le brouillard ou la pluie.

8. Dispositif selon la revendication 7, **caractérisé en ce que** chacune des chambres ne dépasse pas la longueur de 5 mm dans une dimension.

9. Dispositif selon la revendication 7 ou 8, **caractérisé en ce que** des récipients pour capturer les produits cellulaires se trouvent en dessous des chambres.

10. Dispositif selon l'une quelconque des revendications 7 à 9, **caractérisé en ce que**
a) un tuyau ou une liaison Luer sert de raccord
b) il contient au moins une chambre génératrice de brouillard ou d'aérosol
c) il contient une ou plusieurs buses ou douches avec lesquelles une pluie peut être générée
d) il contient en outre un rotamètre pour la mesure des courants volumiques de l'amenée de gaz, des appareils de mesure de température pour le contrôle d'une répartition de température homogène, des filtres à air pour la filtration stérile de l'air d'amenée et d'évacuation, des appareils de mesure de pression, des filtres collecteurs de brouillard et des récipients de capture de condensat
e) le récipient pour la capture des produits cellulaires est relié à un réservoir de produit qui se trouve en dehors de l'espace d'alimentation.

11. Utilisation du dispositif selon l'une quelconque des revendications 7 à 10 pour la culture de cellules animales en densités élevées pour
a) l'obtention de produits cellulaires, constituants cellulaires, virus ou ingrédients actifs
b) l'obtention de médicaments ou
c) la fabrication de produits diagnostiques.
